# EUROPEAN PATENT SPECIFICATION

(11) **EP 4 324 458 B1**
(45) Date of publication and mention of the grant of the patent: **02.09.2026**
(21) Application number: 23190167.9
(22) Date of filing: 08.08.2023
(51) Int. Cl.: A61K 9/14, A61K 47/32, A61K 47/12, A61K 47/38, A61K 9/10

(54) **TRIPLE STABILIZER SYSTEM**
DREIFACHSTABILISIERUNGSSYSTEM
SYSTÈME DE STABILISATION TRIPLE

(30) Priority: 15.08.2022 EP 22190372; 29.03.2023 EP 23164885
(43) Date of publication of application: 21.02.2024
(73) Proprietor: Bayer Aktiengesellschaft, 51373 Leverkusen (DE)
(72) Inventor: Dobrowolski, Adrian, 42369 Wuppertal (DE); Kersten, Elisabeth, 42105 Wuppertal (DE); Ostendorf, Michael, 51375 Leverkusen (DE); Hoheisel, Werner, 51061 Köln (DE); Nüboldt, Christoph, 50969 Köln (DE)
(74) Representative: BIP Patents

(56) References cited:
- WO-A1-2014/009436
- WO-A1-2021/156223
- CERDEIRA ANA M ET AL: "Formulation and drying of miconazole and itraconazole nanosuspensions", INTERNATIONAL JOURNAL OF PHARMACEUTICS, ELSEVIER, NL, vol. 443, no. 1, 4 January 2013 (2013-01-04), pages 209 - 220, XP028979329, ISSN: 0378-5173, DOI: 10.1016/J.IJPHARM.2012.11.044
- BITTERLICH A ET AL: "Process parameter dependent growth phenomena of naproxen nanosuspension manufactured by wet media milling", EUROPEAN JOURNAL OF PHARMACEUTICS AND BIOPHARMACEUTICS, ELSEVIER SCIENCE PUBLISHERS B.V., AMSTERDAM, NL, vol. 92, 9 March 2015 (2015-03-09), pages 171 - 179, XP029226830, ISSN: 0939-6411, DOI: 10.1016/J.EJPB.2015.02.031

## Description

Stabilizer systems are commonly used in the wet comminution of a poorly soluble active ingredients for the production of nanosuspensions, i.e. suspensions in which the active ingredient is present as particles in the submicron range (d90 <1 µm). Apart from stabilizing the poorly soluble active ingredients during production of nanosuspensions, the stabilizer systems are also often required to stabilize these active ingredient nanoparticles in the nanosuspensions themselves. Generally, employed are water-soluble polymers for steric stabilization and charged (ionic) surfactants for electrostatic stabilization of the active ingredient nanoparticles. For example Li et al. in 2019 (Improved dissolution and oral absorption by co-grinding active drug probucol and ternary stabilizers mixtures with planetary beads-milling method*)* described a probucol suspensions stabilized with HPC, F68 and SDS and found that within certain concentrations of the nonionic surfactant F68 (0%, 0.5%, and 1.0%), the active ingredient crystals decreased with the increase of the content of F68, while extra amount of F68 (2.0%) did not further facilitate the size reduction. Moreover WO2021/156223 A1 describes nanosuspensions with an average d50 of 500 nm or less of methyl (4,6-diamino-2-[5-fluoro-I-(2- fluorobenzyl)-IH pyrazolo[3,4-b]pyridin-3-yl]pyrimidin-5-ylIcarbamate in a dispersing agent with one or more stabilizers such as polyvinylpyrrolidone (PVP), sodium dodecylsulfate (SDS), vinylpyrrolidone-vinyl acetate copolymer, ethylene oxide-propylene oxide block copolymer, hydroxypropylmethylcellulose (HPMC), polysorbate, hydroxypropylcellulose (HPC), polyoxyl- 35 castor oil, polyoxyl 15 hydroxystearate, Na-desoxycholate, and combinations thereof. For economic reasons, a high concentration of active ingredients in nanosuspensions is preferred. In order to generate nanosuspensions with a high concentration of active ingredient a higher concentration of the stabilizing polymer has to be used as well. However, there is a concentration limitation for certain stabilizing polymers, either because their solubility in water is too low or because the viscosity becomes too high for comminution at higher polymer concentrations. For example, cellulose derivatives such as hydroxypropyl cellulose (HPC), which can be used as stabilizing polymer, lead to relatively high viscosities even at low concentrations, making processing of active ingredients with HPC as stabilizing polymer via comminution challenging or even impossible. Thus, generally the concentration of the active ingredient had to be reduced if HPC was used as stabilizing polymer, thereby also reducing the concentration of the stabilizing polymer until a viscosity was reached that allowed processing and/or that allowed stabilization of the active ingredient nanoparticles in the nanosuspensions. Accordingly, active ingredients that are preferably stabilized with HPC could only be produced as a lower concentrated nanosuspension. Consequently, compared to higher concentrated suspensions, several batches had to be milled to produce a required amount of active ingredient nanoparticles. Therefore, there was a need to provide higher concentration suspensions of substances stabilized with HPC.

This need is met by providing a composition comprising
- a substance of a particle size between 1 µm and 100 µm and a solubility in water between 0.0001 g/l and 10 g/l
- at least 2 wt % of at least one cellulose based polymer
- at least one anionic surfactant,
- at least one non-ionic surfactant
- wherein the non-ionic surfactant is present in an amount of non-ionic surfactant to at least one cellulose based polymer in the range of between 10: 1 and 1: 10
- wherein the ratio of the at least one cellulose based polymer and the at least one non-ionic surfactant together to the substance is between 2:1 to 1:2,
- and wherein the concentration of the substance of a particle size between 1 µm and 100 µm and a solubility in water between 0.0001 g/l and 10 g/l is in the range of 10,1 wt% to 15 wt% and wherein the least one cellulose based polymer is selected from hydroxymethylcellulose (HPMC) and hydroxypropylcellulose (HPC), the at least one anionic surfactant is selected from sodium dodecyl sulfate (SDS), sodium docusate (dioctyl sodium sulfosuccinate) and sodium oleate and the at least one non-ionic surfactant is a polyvinylpyrrolidone

### BRIEF DESCRIPTION OF THE DRAWINGS

FIG 1 shows that a composition comprising a triple stabilizer system has a lower viscosity than a composition comprising two stabilizers.
FIG 2 shows that via adding PVP K12 to a composition as described herein the viscosity was reduced until a plateau was reached. With further addition of PVP K12 the viscosity was increased again. The upper graph (triangles) represents a composition comprising 3 wt% HPC and 0.2 wt% SDS whereas the lower graph (circles) represents a composition comprising 2 wt% HPC and 0.2 wt% SDS.
FIG. 3 shows that low molecular weight non-ionic surfactant PVPK12 or high molecular weight non-ionic surfactant PVPK25 resulted in a reduction of viscosity. The upper graph (triangles) represents a composition comprising 2 wt% HPC, 0.2 wt% SDS and PVP K25 whereas the lower graph (circles) represents a composition comprising 2 wt% HPC, 0.2 wt% SDS and PVP K12.
FIG. 4 shows that addition of 1wt% to 4 wt% of PVPK12 causes reduction of viscosity of the HPMC-SDS solution.
FIG. 5 shows that the polymer influences the critical micelle concentration of a surfactant i.e. that the viscosity is influenced when a surfactant is employed that, from literature, is known to interact favorably with the used polymer.
FIG 6 shows the resulting particle size distribution after milling of composition A (10.5 wt% active ingredient)
FIG. 7 shows that high active ingredient compositions comprising nano-particles i.e. nanosuspensions are stable even when diluted.
FIG. 8 shows that with increasing HPC content in a system with only two stabilizers (SDS and HPC) the viscosity increases linearly.

### DEFINITIONS

As used herein, the term "particle" refers to a solid, gel, or semisolid material having a relatively small size and characterized in that as said particles have a solubility of less than 10g/l in a suspension comprising water, matrix forming polymer(s) and optionally surfactants as determined e.g. by HPLC.

As used herein the term "micro-particle" which is used synonymously with microparticle refers to particles having an average particle size of between > 999 nm to 100µm.

Preferably the employed micro-particles have an average d90 of between 2µm and 50µm, more preferably between 2µm and 30µm, most preferably between 2µm and 20µm.

As used herein the term "nano" refers to particles having an average particle size - i.e. d50 of the distribution - of ≥ 10 nm to ≤ 999 nm.

Herein the nanoparticles have a particle size distribution of d90 < 2µm, preferably < 1µm, especially preferred < 500 nm and d50 < 1 µm, preferably < 500 nm, especially preferred < 200 nm. The particle size distribution can be determined by methods known in the art such as laser diffraction or Dynamic Light Scattering (DLS). The term d50 or d90 means that 50% or 90% of the total product volume consists of particles smaller than the given value for d50 or d90. The given particle size corresponds to the hydrodynamic particle size when determined by DLS (dynamic light scattering), laser diffraction or electron microscopy and for all methods an equivalent size for spheres is given and meant here. A person skilled in the art is capable of choosing technical equipment suitable for achieving a respective d50 or d90 value. For example it is clear to a skilled person that using an agitated ball mill will result in a smaller d50 and d90 values than using a planetary ball mill, especially if the agitated ball mill is cooled.

If Laser diffraction was used to determine the particle size said laser diffraction was performed in a Malvern Mastersizer 3000. Suspensions (nano- and micro-) were directly injected into the storage tank of Master sizer 3000 without addition of further excipients. The Mastersizer 3000 was equipped with a Hydro MV unit, where deionized water was provided to disperse and dilute the sample further. The dispersed sample was added to the Hydro MV unit until the obscuration reached 5%. The mixer of the Hydro MV unit was set to 2500 rpm. The first three measurements were performed with red and blue laser lights using the Mie light scattering model. Three measurements were performed without ultrasonic.

As used herein the term "active ingredient" refers to an agent, active ingredient, compound, substance, compositions, or mixtures thereof, that provides a pharmacological and/or agrochemical effect.

As used herein the term "critical micelle concentration" (CMC) refers to the minimum concentration of surfactants which is necessary to form a colloidal system, in particular micelles, in a self-assembly process. The critical micelle concentration can be determined as described in A. Fernández, N. González, W. Iglesias, and L. Montenegro (1997) Determination of Critical Micelle Concentration of Some Surfactants by Three Techniques, J. Chemical Education, 74:1227-1231.) Preferably the CMC is measured via surface tension measurements with a tensiometer. Surfactant solutions with different concentrations are prepared and characterized with tensiometer as described in Masouk et al. 2022. Before CMC the surface tension is decreasing with increasing surfactant concentration. At CMC the surface tension is no longer reduced with increasing surfactant concentration.

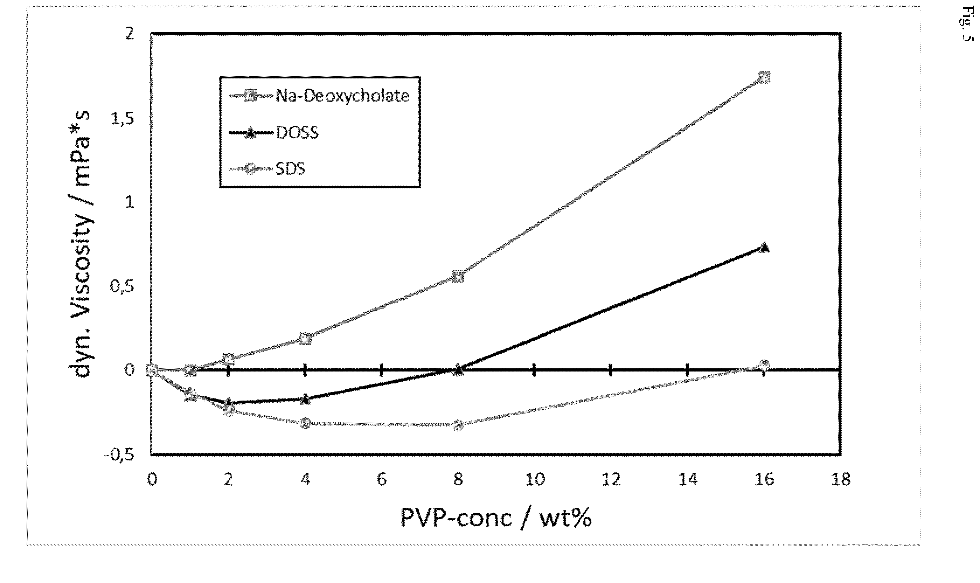

Fig. 5 shows that surfactants which show no reduction of CMC (indicator for poor interaction with polymer) with PVP in the system (Na-Deoxycholate) do not influence the viscosity reduction of the triple-stabilizer-system. Hence a skilled person can determine whether a given surfactant is suitable for the composition described herein.

Hence what is described herein relates also to a composition comprising
- a substance of a particle size between 1 µm and 100 µm and a solubility in water between 0.0001 g/l and 10 g/l
- at least 2 wt % of at least one cellulose based polymer
- at least one anionic surfactant, wherein the critical micelle concentration of the at least one anionic surfactant is influenced by the at least one cellulose based polymer or non-ionic surfactant(Indicator for good interaction between polymer/non-ionic surfactant and anionic surfactant
- at least one non-ionic surfactant
- wherein the non-ionic surfactant is present in an amount of non-ionic surfactant to at least one cellulose based polymer in the range of between 10: 1 and 1: 10
- wherein the ratio of the at least one cellulose based polymer and the at least one non-ionic surfactant together to the substance is between 2:1 to 1:2,
- and wherein the concentration of the substance of a particle size between 1 µm and 100 µm and a solubility in water between 0.0001 g/l and 10 g/l is in the range of 10.1 wt% to 15 wt% and wherein the least one cellulose based polymer is selected from hydroxymethylcellulose (HPMC) and hydroxypropylcellulose (HPC), the at least one anionic surfactant is selected from sodium dodecyl sulfate (SDS), sodium docusate (dioctyl sodium sulfosuccinate) and sodium oleate and the at least one non-ionic surfactant is a polyvinylpyrrolidone

As used herein the term "non-ionic surfactant " refers to surfactants that do not have any ions. The non-ionic surfactant sterically stabilizes the nano-particles and is hence also referred to as steric stabilizer.

### DETAILED DESCRIPTION

As specified above according to a first aspect what is described herein relates to a composition comprising
- a substance of a particle size between 1 µm and 100 µm and a solubility in water between 0.0001 g/l and 10 g/l
- at least 2 wt% of at least one cellulose-based polymer
- at least one anionic surfactant,
- at least one non-ionic surfactant
- wherein the non-ionic surfactant is present in an amount of non-ionic surfactant to at least one cellulose based polymer in the range of between 10: 1 and 1: 10
- wherein the ratio of the at least one cellulose based polymer and the at least one non-ionic surfactant together to the substance is between 2:1 to 1:2,
- and wherein the concentration of the substance of a particle size between 1 µm and 100 µm and a solubility in water between 0.0001 g/l and 10 g/l is in the range of 10.1 wt% to 15 wt% and wherein the least one cellulose based polymer is selected from hydroxymethylcellulose (HPMC) and hydroxypropylcellulose (HPC), the at least one anionic surfactant is selected from sodium dodecyl sulfate (SDS), sodium docusate (dioctyl sodium sulfosuccinate) and sodium oleate and the at least one non-ionic surfactant is a polyvinylpyrrolidone

It is clear to a skilled person, that the remaining weight percentage proportion of the composition described herein is water. Preferably the water is distilled water and/or sterile water.

It was found that via adding the non-ionic surfactant to generate a triple stabilizer system, the viscosity of an aqueous composition comprising at least 2 wt% of a cellulose based polymer and a non-ionic surfactant in an amount of non-ionic surfactant to at least one cellulose based polymer in the range of between 10:1 and 1:10 before and after wet comminution was reduced. This was surprising as it had been expected that adding a further non-ionic surfactant would further increase the viscosity. Therefore, via adding the non-ionic surfactant to generate a triple stabilizer system, it became possible to increase concentration of the at least one cellulose-based polymer while still maintaining the viscosity in a range suitable for comminution/milling. Due to the higher cellulose based polymer concentration also concentration of the substance with a solubility in water between 0.0001 g/l and 10 g/l could be increased. Thus, employing the non-ionic surfactant resulted in a more economic process since the comminution has to be repeated less times. In addition, it was found that via adding the non-ionic surfactant to generate a triple stabilizer system supporting a higher concentration of the substance ensuring a ratio of the at least one cellulose based polymer and the at least one non-ionic surfactant together to the substance between 2:1 to 1:2 the particles size distribution (PSD) of the nanoparticles derived from the composition described herein remained almost exactly constant after drying and redispersing compared to the PSD of the nanoparticles derived from the composition described herein prior to drying.

Viscosity can be measured for example via rotational viscosimeter, capillary viscosimeter, falling sphere viscosimeter or dip cup viscosimeter which all are established methods and well known to a person skilled in the art. An example for a rotational viscosimeter is the modular compact rheometer MCR102e by Anton Paar. For flow and viscosity curves measured via rotational viscometry, a shear rate range is specified, and the viscosity is measured as a function of the shear rate. In the case of the flow curve, the shear stress is usually shown on the y-axis and the shear rate on the x-axis; in the case of the viscosity curve, the viscosity is shown on the y-axis and the shear rate on the x-axis. (Rotational viscometry :: Anton Paar Wiki (anton-paar.com).

In a preferred embodiment of the composition the substance of a particle size between 1 µm and 100 µm and a solubility in water between 0.0001 g/l and 10 g/l is an active ingredient hence the composition described herein comprises in an aqueous suspension
- an active ingredient of a particle size between 1 µm and 100 µm and a solubility in water between 0.0001 g/l and 10 g/l
- at least 2 wt% of at least one cellulose-based polymer
- at least one anionic surfactant,
- at least one non-ionic surfactant
- wherein the non-ionic surfactant is present in an amount of non-ionic surfactant to at least one cellulose based polymer in the range of between 10: 1 and 1: 10
- wherein the ratio of the at least one cellulose based polymer and the at least one non-ionic surfactant together to the active ingredient is between 2:1 to 1:2,
- and wherein the concentration of the active ingredient of a particle size between 1 µm and 100 µm and a solubility in water between 0.0001 g/l and 10 g/l is in the range of 10.1 wt% to 15 wt%.

A skilled person is aware of the fact that the composition described herein could also comprise more than one substance e.g. a mixture of two active ingredients. However, in practice this embodiment seems unlikely due to regulatory requirements regarding active ingredients.

The cellulose-based polymer is selected from hydroxymethylcellulose (HPMC) and hydroxypropylcellulose (HPC).

In an especially preferred embodiment HPC is used as cellulose based polymer. In a further preferred embodiment the composition comprises HPC in a range of between 2 wt% and 3 wt%.

The anionic surfactant is chosen from the group consisting of sodium dodecyl sulfate (SDS), sodium docusate (dioctyl sodium sulfosuccinate) and/or sodium oleate.

In an especially preferred embodiment the anionic surfactant is sodium dodecyl sulfate (SDS).

When employing an anionic surfactant the composition described herein also has beneficial effects for the production of redispersible powders as described in WO2021069350A1. To produce re-dispersible powders after drying of these nanosuspensions, it was shown in WO2021069350A1 that a drug to polymer ratio of ~1:1 is advantageous. By redispersible powders it is meant that these powders, with the addition of the at least equal amount of water removed during drying, form a suspension with a size distribution where again the d90 is < 1µm. If the suspension has a higher concentration less water has to be evaporated during drying to obtain the desired amount of redispersible powder (WO2021069350A1).

To a person skilled in the art it is clear that also a surfactant other than an anionic surfactant can be used. The surfactant can be selected from any surfactant (i.e. charged or non-charged) as long as the critical micelle concentration of the surfactant is influenced by the cellulose based polymer (indicator for good interaction between anionic surfactant and used polymers / nonionic surfactants). If this is the case than the surfactant is suitable.

Surfactants known to a skilled person include charged surfactant selected from acylamino acids (and salts thereof), such as: acylglutamates, for example sodium acylglutamate, di-TEA-palmitoylaspartate and sodium acaprylglutamate; acylpeptides, for example palmitoyl hydrolyzed milk protein, sodium cocoyl hydrolyzed soy protein and sodium/potassium cocoyl hydrolyzed collagen; Sarcosinates, for example, myristoyl sarcosine, TEA-lauroyl sarcosinate, sodium lauroyl sarcosinate and sodium cocoyl sarcosinate; Taurates, for example, sodium lauroyl taurate and sodium methyl cocoyl taurate; acyl lactylates, luroyl lactylate, caproyl lactylate, alaninates; carboxylic acids and derivatives, such as: carboxylic acids, for example lauric acid, aluminum stearate, magnesium alkanolate and zinc undecylenate; ester carboxylic acids, for example calcium stearoyl lactylate and sodium PEG lauramide carboxylate; Ether carboxylic acids, for example sodium laureth carboxylate and sodium PEG cocamide carboxylate; phosphoric acid esters and salts, such as DEA-oleth-phosphate and dilaureth-phosphate; sulfonic acids and salts, such as acyl-isethionates, e.g. sodium/ammonium cocoyl isethionate, alkyl aryl sulfonates, alkyl sulfonates, for example sodium coco monoglyceride sulfate, sodium C-olefin sulfonate, sodium lauryl sulfoacetate and magnesium PEG cocamide sulfate, sulfosuccinates, for example dioctyl sodium sulfosuccinate, disodium laureth sulfosuccinate, disodium lauryl sulfosuccinate and disodium undecylenamido MEA sulfosuccinate; as well as sulfuric acid esters, such as alkyl ether sulfate, for example sodium, ammonium, magnesium, MIPA, TIPA laureth sulfate, sodium myreth sulfate and sodium C-pareth sulfate, alkyl sulfates, for example sodium, ammonium and TEA lauryl sulfate,, alkylamines, alkylimidazoles, ethoxylated amines, quaternary surfactants, esterquats and amphoteric surfactants such as Acyl/dialkyl ethylenediamines, for example sodium acylamphoacetate, disodium acylamphodipropionate, disodium alkylamphodiacetate, sodium acylamphohydroxypropyl sultonate, disodium acylamphodiacetate and sodium acylamphopropionate, and N-alkylamino acids, for example, aminopropylalkylglutamide, alkylaminopropionic acid, natuium alkylimidodipropionate and lauroamphocarboxyglycinate.

Examples of non-ionic surfactants are polyvinylpyrrolidone-vinylacetate-copolymer, polymethracrylate-derivates, and polyvinylpyrrolidone (PVP). methylcellulose, hydroxymethylcellulose, hydroxyethylellulose, hydroxypropylcellulose, hydroxybutylcellulose, hydroxyethylmethylcellulose, hydroxypropylmethylcellulose, carboxymethylcellulose, sodiumcarboxymethylcellulose, carboxymethylethylcellulose, carboxyalkylcellulose ester, starches, sodium carboxymethylamylopectin, chitosan, dextran sulfate sodium salt, alginic acid, alkali metal and ammonium salts of alginic acid, carrageenans, galactomannans, tragacanth, agar-agar, gum arabic, guar gum, xanthan gum, polyacrylic acid and its salts, polymethacrylic acid and its salts, polyvinyl alcohol, polyvinylpyrrolidone, polyethylene oxide, polypropylene oxide, copolymers of ethylene oxide and propylene oxide, N-vinylpyrrolidone-vinyl acetate copolymers or a mixture of at least two of the aforementioned polymers. Polyvinylpyrrolidones (especially K12 and K30 types) and N-vinylpyrrolidone-vinyl acetate copolymers are particularly preferred. Preferably, the polymer can be a copolymer of ethylene oxide and propylene oxide, in particular a poloxamer, or a polyvinylpyrrolidone, in particular PVPK30.

In a further preferred embodiment the non-ionic surfactant is characterized by a molecular weight between 2,000 g/mol and 16,000g/mol preferably between 3,000 g/mol and 8,000 g/mol and is termed a "low molecular weight non-ionic surfactant ".

In a further preferred embodiment the non-ionic surfactant is characterized by a molecular weight between 18.000 g/mol and 50.000g/mol preferably between 22.000 g/mol and 44.000 g/mol and termed a "high molecular weight non-ionic surfactant ".

In another preferred embodiment the composition comprises 0.01 wt% to 10 wt% low molecular weight non-ionic surfactant or 0.01 wt% to 5wt% high molecular weight non-ionic surfactant .

Polyvinylpyrrolidone is used as non-ionic surfactant .

In an especially preferred embodiment the low molecular weight non-ionic surfactant is PVP K12.

In a preferred embodiment of the composition the non-ionic surfactant is present in an amount of non-ionic surfactant to at least one cellulose based polymer in the range of between 4: 1 and 1:4.

In a further preferred embodiment of the composition the ratio of the at least one cellulose based polymer and the at least one non-ionic surfactant together to the substance is between 2:1 to 1:2. In an especially preferred embodiment of the of the composition the ratio of the at least one cellulose based polymer and the at least one non-ionic surfactant together to the substance is 1:1, as this ratio is optimal for the generation of re-dispersible powders.

In another preferred embodiment of the composition the concentration of the substance of a particle size between 1 µm and 100 µm in the range of 10.1 wt% to 12 wt%.

In another preferred embodiment of the composition the solubility of the substance of a particle size between 1 µm and 100 µm in water is in the range of between 0.001g/l and 1 g/l.

In another preferred embodiment the composition consist of at least one active ingredient of a particle size between 1µm and 100µm and a solubility in water between 0.001 g/l and 1 g/l, HPMC as cellulose based polymer, SDS as anionic surfactant, PVP12 as low molecular weight non-ionic surfactant , wherein the non-ionic surfactant is present in an amount of non-ionic surfactant to at least one cellulose based polymer in the range of between 4:1 and 1:4, wherein the ratio of the at least one cellulose based polymer and the at least one non-ionic surfactant together to the substance is 1:1 and wherein the concentration of the active ingredient of a particle size between 1 µm and 100µm and a solubility in water between 0.001 g/l and 10 g/l is in the range of 10.1 wt% to 12 wt%.

In an especially preferred embodiment the composition consist of at least one active ingredient of a particle size between 1µm and 100µm and a solubility in water between 0.001 g/l and 1 g/l, 2 wt% HPC as cellulose based polymer, SDS as anionic surfactant, PVP12 as low molecular weight non-ionic surfactant , wherein the non-ionic surfactant is present in an amount of non-ionic surfactant to at least one cellulose based polymer in the range of between 4:1 and 1:4, wherein the ratio of the at least one cellulose based polymer and the at least one non-ionic surfactant together to the substance is 1:1 and wherein the concentration of the active ingredient of a particle size between 1µm and 100 µm and a solubility in water between 0.001 g/l and 10 g/l is in the range of 10.1 wt% to 12 wt%.

In another especially preferred embodiment the composition consist of at least one active ingredient of a particle size between 1µm and 100µm and a solubility in water between 0.001 g/l and 1 g/l, 3 wt% of HPC as cellulose based polymer, SDS as anionic surfactant, PVP12 as low molecular weight non-ionic surfactant, wherein the non-ionic surfactant is present in an amount of non-ionic surfactant to at least one cellulose based polymer in the range of between 4:1 and 1:4, wherein the ratio of the at least one cellulose based polymer and the at least one non-ionic surfactant together to the substance is 1:1 and wherein the concentration of the active ingredient of a particle size between1µm and 100 µm and a solubility in water between 0.001 g/l and 10 g/l is in the range of 10.1 wt% to 12 wt%.

In a preferred embodiment the composition is further processed to form a re-dispersible powder as described in WO2021069350A1. In detail the composition in this case is further process via nano-grinding thereby providing a composition comprising nano-particles of the substance characterized by a nano-particle size of a d90 between 10 nm and 999 nm as measured by Dynamic Light Scattering (DLS), laser diffraction or electron microscopy.

Suitable methods of nano-grinding (also termed nano grinding, nanogrinding or nanomilling) can be selected from the group consisting of wet bead milling in stirred media mills, wet bead milling in planetary mills - especially suitable for small amounts - and high pressure homogenization. Alternatively, the application of high shear forces in aqueous suspensions like in a high pressure homogenization process or the application of high impact forces between the particles like in a microfluidizer can be used to produce nano-particles.

To a skilled person it is clear that if nano grinding is used for providing the seeding material (i.e. production of nanoscale crystals) said nano grinding can only be performed if the nano-particles are not completely dissolved. Hence the skilled person would choose a suitable temperature within the temperature range of between 253 K and 323 K, preferably between 273 K and 303 K for nano-grinding the nano-particles in the aqueous suspension.

Preferably, if nano-grinding is used, a preferred nano-grinding time is observed during grinding in order to achieve better redispersion of the dried powder containing nanoparticles. This preferred grinding time (t-preferred) is significantly longer than the grinding time that would typically be required to achieve the necessary particle size. Preferably said preferred grinding time (t-preferred) is at least 1.5 times t0, preferably it is at least 2 times t0 and particularly preferably at least 4 times t0. Here, t0 is considered to be the usual grinding time at which the d90 value of the particle size distribution is 1.5 times the d90 value reached after 12 hours and is referred to as d90(12h). In other words, this means that d90 (12h) is 2/3 of the d90 value at the usual grinding time t0. Grinding time is understood to mean the residence time of the suspension in the mill, which explicitly means that the possible residence time of the suspension in an optionally present holding tank is not counted towards the grinding time, i.e. towards the comminution time (tz).

Following nano-grinding the composition is spray dried according to step B) of the method described in WO 2021/069350 and to form a powder and optionally further process to form a tablet. This has the advantage that it can be stored more conveniently.

In addition, as mentioned above it was found that via adding the non-ionic surfactant to generate a triple stabilizer system supporting a higher concentration of the substance and ensuring a ratio of the at least one cellulose based polymer and the at least one non-ionic surfactant together to the substance between 2:1 to 1:2, the particles size distribution (PSD) of the nanoparticles derived from the composition described herein remained almost exactly constant after drying and redispersing compared to the PSD of the nanoparticles derived from the composition described herein prior to drying. In contrast for example the nanosuspension described by Li et al. 2019 does not show an almost exactly constant PSD after redispersing (cf. Example 9).

In other words, drying and redispersion of a composition comprising nanoparticles obtained from the composition described herein in aqueous media that do not bring about complete molecular dissolution of the substance, lead to the particle size distribution (PSD) of the original composition comprising nanoparticles obtained from the composition described herein remaining almost exactly constant hence differs by comparison with the PSD of the redispersed powder only by a factor X within a range from ≥ 1 to ≤ 3, based on the d₉₀ value of the respective suspensions. X here corresponds to the ratio n/v, where n represents the d₉₀ value of the PSD after drying and redispersion and v the d₉₀ value before drying. X is particularly preferably ≥ 1 to ≤ 1.2.

It is especially preferred that if the composition is processed via nano-grinding and spray drying the ratio of the at least one cellulose based polymer and the at least one non-ionic surfactant together to the substance is 1:1.Alternatively to nano-grinding the seeding material (i.e. the nano crystals) can be provided via high pressure homogenization, or antisolvent precipitation.

In another aspect what is described herein relates to a method for producing a composition comprising nano-particles starting from the composition described herein, wherein the method comprises the following steps:
- providing composition as described above comprising in an aqueous suspension a substance of a particle size between 1 µm and 100µm and a solubility in water between 0.0001 g/l and 10 g/l, at least 2 wt % of at least one cellulose based polymer, at least one anionic surfactant and at least one non-ionic surfactant , wherein the at least one non-ionic surfactant is present in an amount of non-ionic surfactant to at least one cellulose based polymer in the range of between 10:1 and 1:10 wherein the ratio of the at least one cellulose based polymer and the at least one non-ionic surfactant together to the substance a particle size between 1µm and 1 nm is between 2:1 to 1:2, and wherein the concentration of the substance in the range of 10.1 wt% to 15 wt%.
- comminution of the composition for a time span until the particle size stays constant in a temperature range between 253K and 323 K thereby generating the composition comprising nano-particles.

Preferably this aspect relates to a method for producing a composition comprising nano-particles starting from the composition described herein, wherein the method comprises the following steps:
a) providing composition as described above comprising an active ingredient of a particle size between 1 µm and 100µm and a solubility in water between 0.0001 g/l and 10 g/l, at least 2wt% of at least one cellulose based polymer, at least one anionic surfactant and at least one non-ionic surfactant , wherein the at least one non-ionic surfactant is present in an amount of non-ionic surfactant to at least one cellulose based polymer in the range of between 10: 1 and 1: 10 wherein the ratio of the at least one cellulose based polymer and the at least one non-ionic surfactant together to the substance a particle size between 1µm and 1 nm is between 2:1 to 1:2, and wherein the concentration of the substance in the range of 10.1 wt% to 15 wt%.
b) comminution of the composition for a time span until the particle size stays constant in a temperature range between 253K and 323 K thereby generating the composition comprising nano-particles
c) drying the mixture comprising nanoparticles obtained in step B)
d) re-dispersing the powder obtained in step D)

As mentioned above this method and the resulting re-dispersible powder have the advantage that a high concentration of active ingredient can be processes and the particles size distribution (PSD) of the nanoparticles remains almost exactly constant after drying and redispersing compared to the PSD of the nanoparticles derived from the composition described herein prior to drying.

In yet another aspect what is described herein relates to a composition comprising
- a substance of a particle size between 1 nm and 999 nm and a solubility in water between 0.0001 g/l and 10 g/l
- at least 2wt % of at least one cellulose based polymer
- at least one anionic surfactant,
- at least one non-ionic surfactant ,
- wherein the non-ionic surfactant is present in an amount of non-ionic surfactant to at least one cellulose based polymer in the range of between 10: 1 and 1: 10
- wherein the ratio of the at least one cellulose based polymer and the at least one non-ionic surfactant together to the substance is between 2:1 to 1:2,
- and wherein the concentration of the substance of a particle size between 1µm and 100µm and a solubility in water between 0.0001 g/l and 10 g/l is in the range of 10.1 wt% to 15 wt% for use as a medicament.

Said composition for use as a medicament is preferably a nano-suspension. Said composition is preferably manufactured via the method for producing a composition comprising nano-particles described above.

### EXAMPLES

### 1. Viscosity of a triple stabilizer system compared to a composition comprising two stabilizers

In this example two compositions were compared. Composition A was an aqueous i.e. water-based suspension comprised as substance an active ingredient (10.5 wt%, here Rivaroxaban), as the at least one cellulose-based polymer hydroxypropylcellulose (HPC) 2 wt%, as the at least one anionic surfactant a charged surfactant here SDS (0.2% SDS) and as the at least one non-ionic surfactant a low molecular weight non-ionic surfactant PVP K12 (8 wt%) and 79.3 wt% water.

Composition B comprised 10.5 wt % active ingredient (here Rivaroxaban), 10 wt % HPC and 0.2 wt % SDS. The components of both compositions were stirred on a magnetic stirrer at room temperature.

Viscosity was measured with rotational viscometry with a modular compact rheometer (MCR) 302e (Anton Paar). A concentric cylinder geometry was used. Shear rate was varied logarithmic from 0.1 1/s to 1000 1/s. 6 measurement points per decade were used. The measurement time was varied form 10s for a shear rate of 0.1 1/s and 1s for 1000 1/s. Shear rate vs. Shear stress was plotted. The slope described the viscosity. The shear rate independent viscosity was evaluated, and the middle value was used.

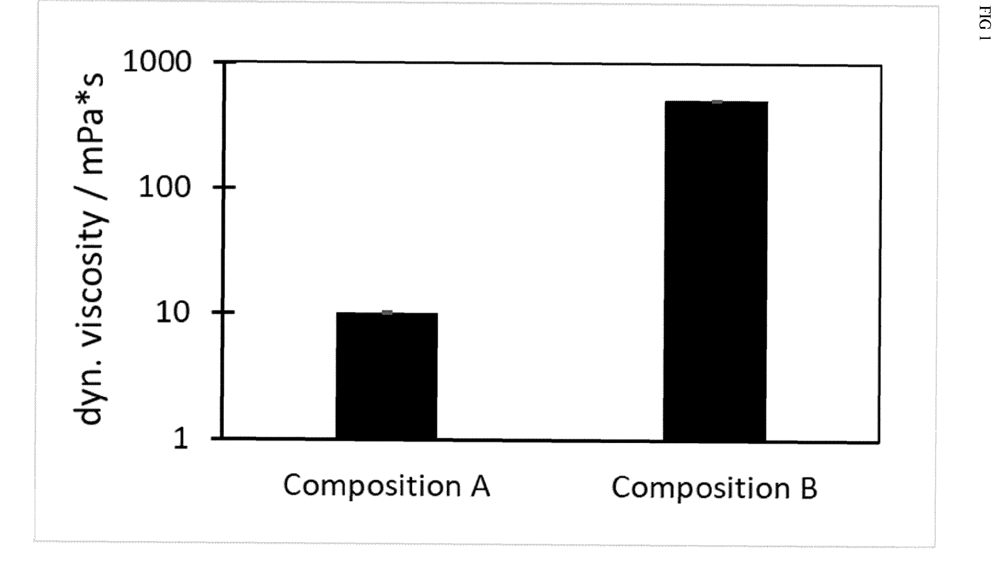

As depicted in Fig. 1 it was found that composition A (API-HPC-PVPK12-SDS) had a lower viscosity than composition B (API-HPC-SDS).

### 2. High active ingredient nanosuspension

Furthermore, the viscosity of composition B was so high, that it was not possible to separate the milling beads from the high viscous composition B during wet milling. Hence it was not practical to process composition B into nanosuspension via wet milling. The processing of composition A via wet milling on the other hand was unproblematic and resulted in a stable high active ingredient nanosuspension. Separation of grinding beads from the nanosuspension was possible.

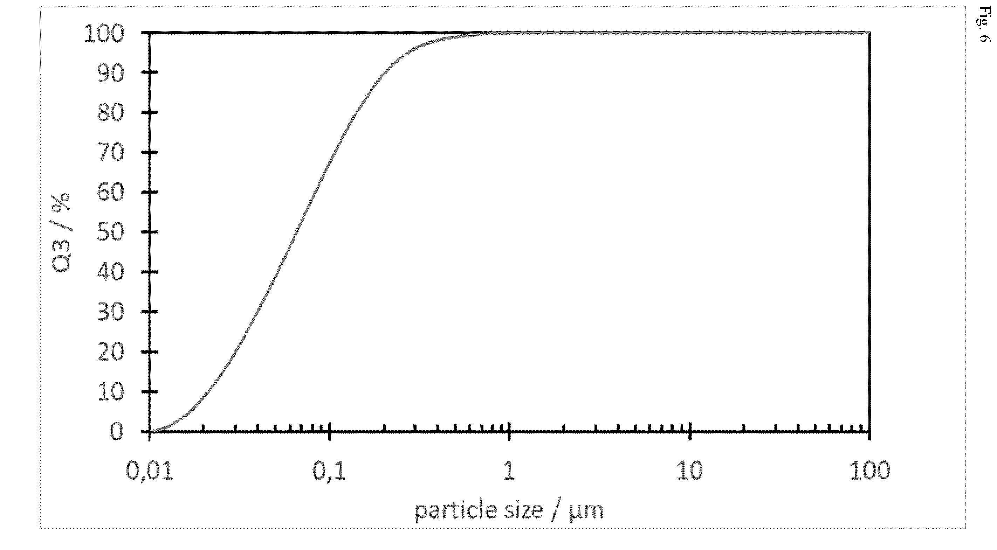

To evaluate whether a high active ingredient nano-suspension could also be generated using only a anionic surfactant here SDS and a low molecular weight non-ionic surfactant PVP K12 composition C comprising 10.1 wt% API (here Rivaroxaban), 10wt% PVPK12 and 0.2 wt% SDS and 79.7 wt% water. was produced and processed into an aqueous nanosuspension. The resulting aqueous nano-suspension was instable after dilution and therefore unsuitable for further applications (Data not shown). Fig. 6 shows the resulting particle size distribution after milling of composition A (10.5 wt% active ingredient). Milling conditions were used as described in example 8.

The results of composition A were repeated with two other active ingredients (data not shown)

### 3. Influence of non-ionic surfactant (PVP) concentration

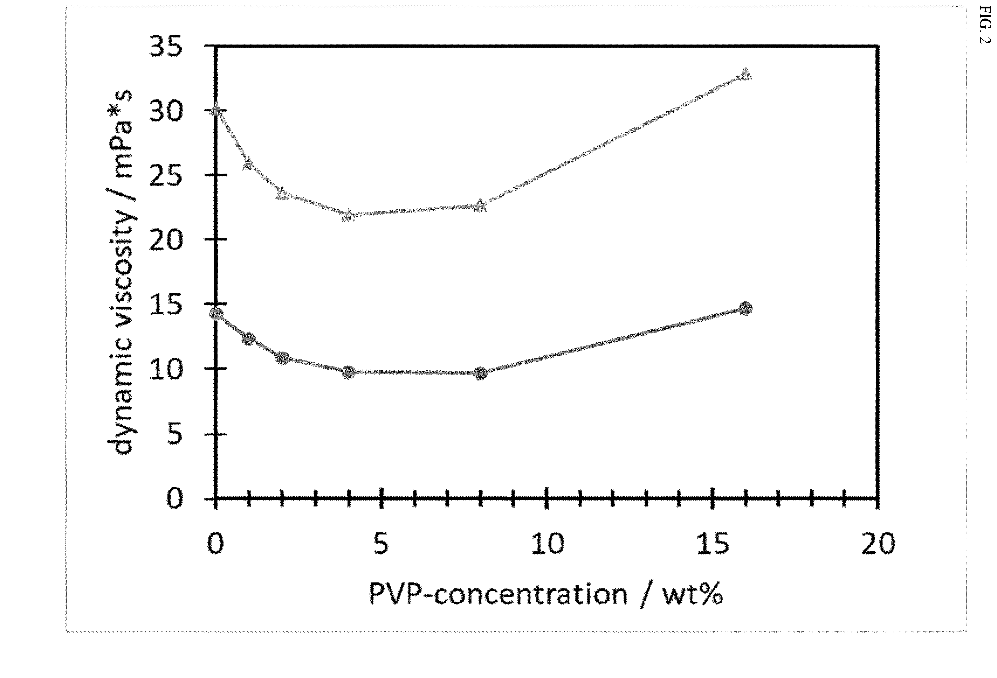

Moreover, it was found that with increasing PVP K12 content the viscosity was reduced until a plateau was reached. With further addition of PVP K12 the viscosity increased again (Fig. 2). Viscosity was measured as described in example 1. Used were two compositions in aqueous suspension one composition comprising 3 wt% HPC and 0.2 wt% SDS and 96.8 wt% water whereas the lower graph (circles) represents a composition comprising 2 wt% HPC and 0.2 wt% SDS and 97.8 wt% water.

**Table 2**

| **PVPK12** | **HPC 2 wt%** | **HPC 3wt%** |
|---|---|---|
| 0.0 | 14.29 | 30.23 |
| 0.5 | 13.42 | no data point |
| 1.0 | 12.36 | 25.94 |
| 2.0 | 10.90 | 23.65 |
| 4.0 | 9.78 | 21.94 |
| 8.0 | 9.68 | 22.72 |
| 16.0 | 14.68 | 32.87 |

Table 2 demonstrates that with increasing PVP K12 content the viscosity was reduced until a plateau was reached. With further addition of PVP K12 the viscosity increased again.

### 4. Influence of non-ionic surfactant other than PVP 12

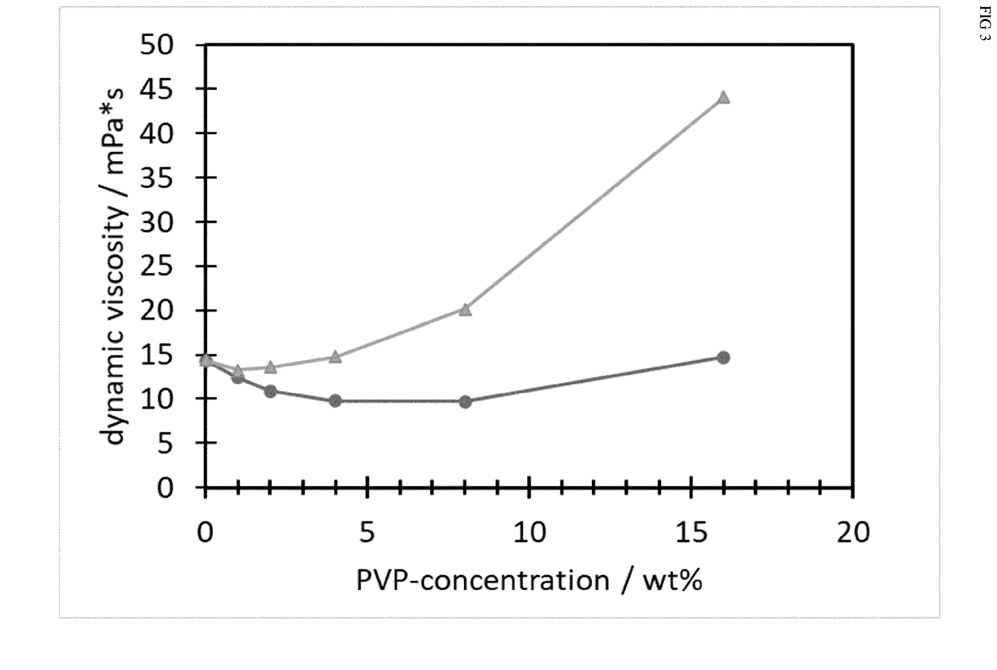

In this example HPC-SDS solutions (2wt% and 0.2 wt%) in water with varied amount of PVPK12 or PVPK25 were produced for a viscosity study. SDS and HPC content were kept constant (2 wt% HPC and 0.2 wt% SDS) and the PVPK12/PVPK25 content was varied. Fig 3 shows that low molecular weight PVP - here PVP K12 - was suitable for viscosity reduction of a HPC-SDS solution at least up to a value of 8 wt%. Also, addition of the high molecular weight non-ionic surfactant PVPK25 resulted in a reduction of viscosity if added up to 5wt%. Viscosity was measured as described in example 1.

### 5. Influence of cellulose-based polymer other than HPC

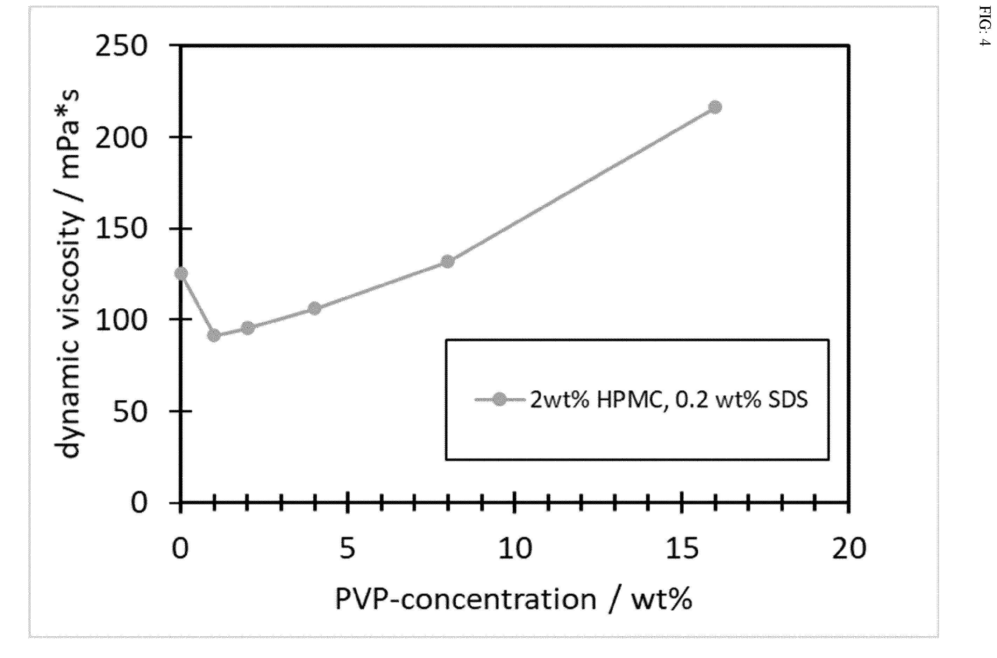

In this example HPC was replaced by HPMC to produce Cellulose-SDS solutions in water with different amount of PVPK12 for a viscosity study. SDS (0,2wt%) and HPMC (2wt%) content were kept constant and the PVPK12 content was varied in the range of 0-15 wt%. Fig 4 shows that addition of 1wt% to 4 wt% of PVPK12 still cause reduction of viscosity of the HPMC-SDS solution. Viscosity was measured as described in example 1.

### 6. Influence of anionic surfactants other than SDS

In this example HPC-PVP solutions in water with different anionic surfactants Na-Docusate (DOSS), Na-Deoxycholate and SDS were produced. HPC concentration was constant at 2wt%, anionic surfactant concentration was constant at 0.2wt%. PVPK12 concentration was varied from 0 to 16 wt%. Viscosity was measured as described in example 1. For better comparability of samples, the relative viscosity change was calculated and plotted against PVPK12 concentration. $Δ {η}_{rel} = \frac{{η}_{n} - {η}_{0}}{{η}_{0}}$ was used for calculation of relative viscosity change. *η*₀ represents the viscosity of the respective cellulose derivative anionic surfactant solution without PVP and *ηₙ* the respective viscosity of the cellulose derivative anionic surfactant solutions after PVP has been added. If *ηₙ* = *η*₀ the resulting relative viscosity change is zero. Fig 5 shows the impact of anionic surfactant on the viscosity reduction induced by PVPK12 addition. SDS (circles, lowest curve) has the strongest impact followed by DOSS (middle curve, triangles). Na-Deoxycholate (top curve, squares) containing solutions do not show a reduction of viscosity when PVPK12 is added. It is literature known that Na-Deoxycholate does not have a good interaction with PVP or cellulose derivates (Majhi, 2000, physicochemical investigations on the interaction of surfactants and salts with polyvinylpyrrolidone in aqueous medium). In other words the data shows that the viscosity was not influenced by nonionic surfactant PVP when an anionic surfactant with poor interaction with used non-ionic surfactant and the cellulose derivates (i.e. the polymer) was employed suggesting that nonionic surfactant PVP/ cellulose derivate HPC solution did not influence the CMC of the anionic surfactant Na-Deoxycholate. Indicator for poor interaction could be shown with surface tension measurements were the CMC of Na-Deoxycholate was not influenced by used polymers (Majhi, 2000). Hence in this example the viscosity of triple stabilizer system was influenced when the anionic surfactants Na-Docusate (DOSS) and SDS were used. CMC of those ionic surfactants are expected to be influenced by PVP and HPC. Therefore Na-Docusate (DOSS) and SDS were suitable (anionic) surfactants.

### 7. Influence of increasing HPC in a system with two stabilizers

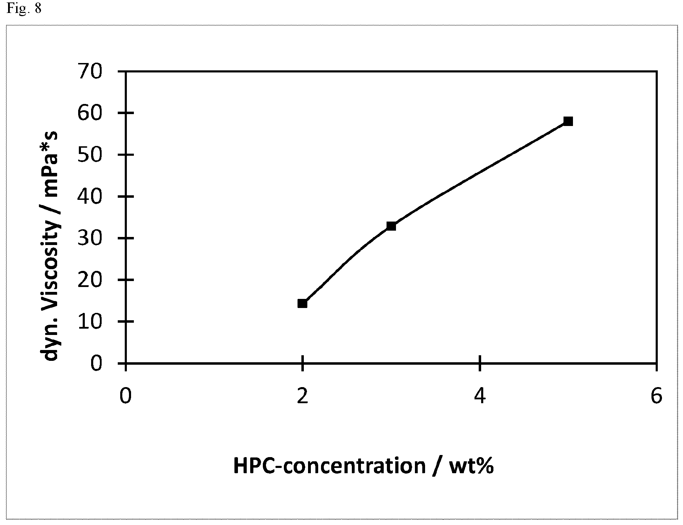

In this example water-based HPC-SDS solutions (i.e. solutions with only two stabilizers) were prepared with constant SDS-concentration (0.2wt%) and varied HPC-concentration. Fig. 8 shows that with increasing HPC content the viscosity increases linearly. Viscosity was measured as described in example 1.

### 8. Dilution experiments

In order to show that the high active ingredient compositions are stable even when diluted as it would be the case if an active ingredient is applied to a subject, different settings were compared In first step aqueous stabilizer solutions were prepared
(1) 10 wt% PVPK12, 0.2 wt% SDS,
(2) 8 wt% PVPK12, 2 wt% HPC and 0.2 wt% SDS and
(3) 2 wt% HPC and 0.2 wt% SDS.

After that API-microparticles were dispersed inside the prepared stabilizer solution. The result were three microsuspensions:
(1) active ingredient (Rivaroxaban) 10 wt% ,10 wt% PVPK12, 0.2 wt% SDS
(2) active ingredient (Rivaroxaban) 10 wt% 8 wt% PVPK12, 2 wt% HPC and 0.2 wt% SDS
(3) active ingredient (Rivaroxaban) 2 wt% 2 wt% HPC and 0.2 wt% SDS

These microsuspensions were the starting material for the preparation of nanosuspensions via stirred media milling. In the next step nano-suspensions were prepared from each of the three microsuspensions via stirred media milling in picoliq by hosokawa alpine. All suspensions were processed at 35°C with 2500-3000 rpm in 90 ml milling chamber until a constant particle size was reached. 80% of milling chamber volume was filled with 0.4-0.6 mm ZrO₂ grinding beads by silibeads.

The dilution of the nano-suspensions was carried out inside the storage tank of Mastersizer 3000. The systems 1.1, 2 and 3 were diluted in 100 ml distilled water at room temperature and constant stirrer speed of 1750 rpm. System 1.2 was diluted with PVPK12 and 0.2 wt% SDS instead of distilled water inside the storage tank of mastersizer 3000. At time point 0, all systems were diluted.

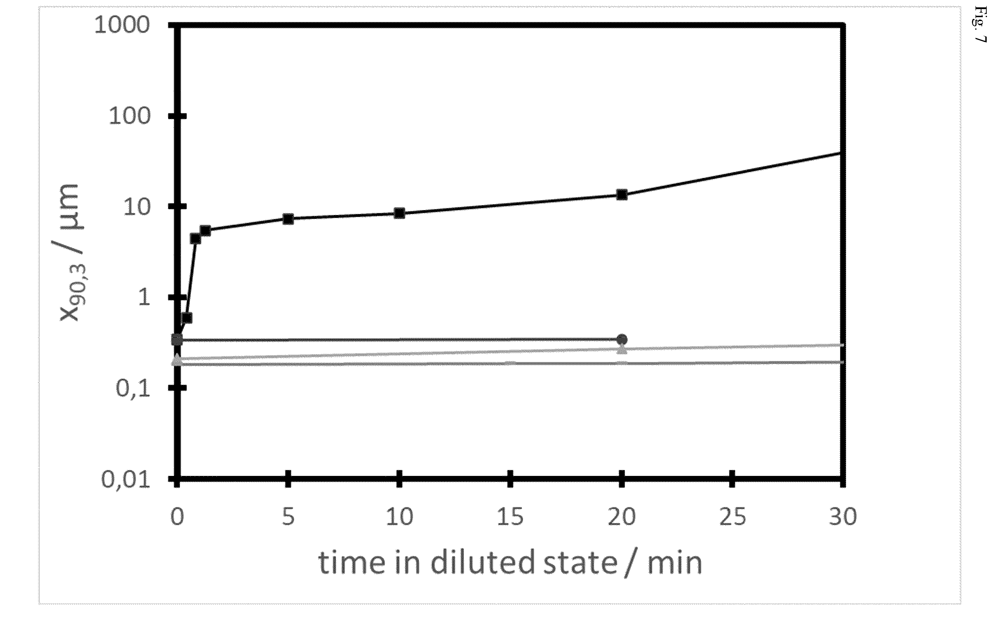

The results are depicted in Fig. 7. The figure shows the x_{90,3} over time i.e. the 90 % of all particles had the depicted size or were smaller as measured by laser diffraction. The top graph (squares) ("system 1.1) represents an active ingredient (Rivaroxaban) 10 wt% nanosuspension which was stabilized with 10 wt% PVPK12 and 0.2 wt% SDS and measured at room temperature in 100 ml distilled water in mastersizer 3000 laser diffractometer by malvern. The top middle graph (circles) ("system 1.2") represents the same suspension but measured in stabilizer solution containing 10wt% PVPK12 and 0.2 wt% SDS instead of distilled water inside the storage tank of Mastersizer 3000. The top bottom graph (triangles) ("system 2") represents an active ingredient nanosuspension (Rivaroxaban) (10 wt%) which was stabilized with 8 wt% PVPK12, 2 wt% HPC and 0.2 wt% SDS. The bottom graph (no marking) ("system 3") represents an active ingredient (Rivaroxaban) 2 wt% nanosuspension which was stabilized with 2 wt% HPC and 0.2 wt% SDS and measured in 100ml distilled water at room temperature in mastersizer 3000 laser diffractometer by malvern. Hence in all cases the SDS concentration (0.2 wt%) was the same.

It was found that the dilution of system 1.1 inside water resulted in an instable composition since the nanoparticles agglomerated and formed larger particles in case of PVP stabilized nanosuspension.

Dilution with PVP in water (system 1.2) did not have this effect because the PVP in solution at the concentration used herein in the dilution medium ensured the stability of the diluted nanoparticles
System 3 did not show any agglomeration during the measurement time. However, it contained only 2wt% active ingredient.

Also, system 2 did not show any agglomeration, even though instead of 2 wt% (system 3), 10wt% API were used. Hence the triple stabilizer system ensured stability during and after dilution. Hence it can be concluded that a triple stabilizer system will also stabilize the active ingredient nano-particles under physiological conditions i.e. during dilution in the gastro-intestinal tract.

### 9. Comparison to ternary stabilizer system

As described above the publication *Li et al. 2019* is known that described the use of an aqueous ternary stabilizer system. However, the ratio of the at least one cellulose based polymer and the at least one non-ionic surfactant together to the substance in Li et al is 6.5: 1 and not between 2:1 to 1:2. To demonstrate that this ratio does not lead to a reduced viscosity and a redispersible powder the following experiment was conducted:
Two composition as given below in Table 1 were generated and the components of both compositions were stirred on a magnetic stirrer at room temperature.

Viscosity was measured with rotational viscometry with a modular compact rheometer (MCR) 302e (Anton Paar). A concentric cylinder geometry was used. Shear rate was varied logarithmic from 0.1 1/s to 1000 1/s. 6 measurement points per decade were used. The measurement time was varied form 10s for a shear rate of 0.1 1/s and 1s for 1000 1/s.

The compositions were spray dried using the Labspraydryer by ProCept with the settings 110°C drying temperature, 0,3 m³/min volume flow rate of the drying gas, 0,4 bar spray nozzle pressure, 5,5 g/min volume flow rate of the composition to be dried at a spray nozzle diameter of 0,8 mm. Afterwards the spray dried material was redispersed in distilled water.

| | Li et al (wt%) | Composition I (wt%) | Composition II (wt%) | Composition of Example 1 (wt%) |
|---|---|---|---|---|
| API | 19.38 wt% | **10.1** wt% (x 1.92 = 19.4) | 10.1 wt% (x 1.92 = 19.4) | 10.5 |
| SDS | 0.78 wt% | 0.2 wt% (x1.92 = 0.34 | 0.2 wt% | 0.2 |
| PVP | 0.78 wt% (F68 not PVP) | **0.404 wt%** (x 1.92 = 0.78) | 0 wt% | 8 |
| HPC | 1.55 wt% | **0.808 wt%** (x 1.92 = 1.55) | 0.808 wt% | 2 |
| Ratio API: (HPC+PVP) | 8.33 | **8.33** | | 10.5:10 = 1.05 |
| Viscosity | | 2.579955 | 2.1306525 | |
| Redispersible after drying With constant Particle size | No | No | | Yes |
| | D90: 0,416 µm (milled) | D90: 1,48 µm (milled) | | D90: 0,145 µm (milled) |
| | | D90: 16,3 µm (SD) | | |
| | D90: 14,4 µm (SD) | | | D90: 0,146 µm (SD) |

It can be seen from Table 1 that the tenary stabilizer system of Li et al. showed a higher viscosity compared to a stabilizer system with only two stabilizers. Therefore the ternary stabilizers system of Li et al. composed of a primary stabilizer (water soluble cellulose derivative, e.g. HPC), a nonionic surfactant (Pluronic ^{®}F68), and an anionic surfactant (SDS) was effective in reduction of particle size, improving dissolution rate and physical stability but not in decreasing viscosity of a composition before and after wet comminution.

Furthermore the viscosity of the following to compositions was measured as described in example 1
1) 0.6 wt% PVP, 1.2 wt% HPC, 0.2 wt% SDS, 98 wt% water
2) 1.2 wt% HPC, 0.2 wt% SDS, 98.6 wt% water

These compositions were calculated from the compositions of Li et al. assuming 15 wt % API instead of 19.38 wt% API as given in Li et al. in Table 1.

The results are shown in Table 3 below:

**Table 3**

| | | |
|---|---|---|
| | 0.6 wt% PVP, 1.2 wt% HPC 0.2 wt% SDS | 1.2 wt% HPC und 0.2 wt% SDS |
| Viscosity In mPa*s | 5.7 | 5.4 |

The results of Table 3 show that a least 2 wt% HPC need to be present for the viscosity reduction of a triple stabilizer system.

## Claims

1. A compositions comprising
• a substance of a particle size between 1 µm and 100 µm as measured by Laser diffraction and a solubility in water between 0.0001 g/l and 10 g/l
• at least 2wt % of at least one cellulose based polymer
• at least one anionic surfactant,
• at least one non-ionic surfactant ,
• wherein the non-ionic surfactant is present in an amount of non-ionic surfactant to at least one cellulose based polymer in the range of between 10: 1 and 1: 10
• wherein the ratio of the at least one cellulose based polymer and the at least one non-ionic surfactant together to the substance is between 2:1 to 1:2,
• and wherein the concentration of the substance of a particle size between 1µm and 100µm and a solubility in water between 0.0001 g/l and 10 g/l is in the range of 10.1 wt% to 15 wt% and
• wherein the least one cellulose based polymer is selected from hydroxymethylcellulose (HPMC) and hydroxypropylcellulose (HPC), the at least one anionic surfactant is selected from sodium dodecyl sulfate (SDS), sodium docusate (dioctyl sodium sulfosuccinate) and sodium oleate and the at least one non-ionic surfactant is a polyvinylpyrrolidone

2. Composition according to claim 1 wherein the composition the substance of a particle size between 1µm and 100µm and a solubility in water between 0.0001 g/l and 10 g/l is an active ingredient.

3. Composition according to claim 1 wherein the cellulose based polymer is hydroxypropylcellulose HPC and is present in an amount in the range of between 2 wt% and 3 wt%.

4. Composition according to claim 1, wherein the composition comprises 0.01 wt% to 10 wt% low molecular weight non-ionic surfactant or 0.01 wt% to 5wt% high molecular weight non-ionic surfactant .

5. Composition according to claim 6, wherein the low molecular weight non-ionic surfactant is PVP K12.

6. Composition according to claim 1 wherein the non-ionic surfactant is present in an amount of non-ionic surfactant to at least one cellulose based polymer in the range of between 4:1 and 1:4 .

7. Composition according to claim 1 wherein the composition consist of at least one active ingredient of a particle size between 1µm and 100µm as measured Laser diffraction and a solubility in water between 0.001 g/l and 1 g/l, at least 2 wt% HPC as cellulose based polymer, SDS as anionic surfactant, PVP12 as low molecular weight non-ionic surfactant , wherein the non-ionic surfactant is present in an amount of non-ionic surfactant to at least one cellulose based polymer in the range of between 4:1 and 1:4, wherein the ratio of the at least one cellulose based polymer and the at least one non-ionic surfactant together to the substance is 1:1 and wherein the concentration of the active ingredient of a particle size between 1µm and 100 µm and a solubility in water between 0.001 g/l and 10 g/l is in the range of 10.1 wt% to 12 wt%.

8. Method for producing a composition comprising nano-particles with the following steps:
• providing a composition according to claim 1 comprising a substance of a particle size between 1µm and 100µm and a solubility in water between 0.0001 g/l and 10 g/l, at least 2 wt% of at least one cellulose based polymer, at least one anionic surfactant and at least one non-ionic surfactant , wherein the at least one non-ionic surfactant is present in an amount of non-ionic surfactant to at least one cellulose based polymer in the range of between 10:1 and 1:10 wherein the ratio of the at least one cellulose based polymer and the at least one non-ionic surfactant together to the substance a particle size between 1µm and 1 nm is between 2:1 to 1:2, and wherein the concentration of the substance in the range of 10.1 wt% to 15 wt%.
• comminution of the composition for a time span until the particle size stays constant in a temperature range between 253K and 323 K) thereby generating the composition comprising nano-particles.

9. A composition manufactured by the method of claim 8 and therefore comprising
• a substance of a particle size between 1 nm and 999 nm as measured by Dynamic Light Scattering (DLS) and a solubility in water between 0.0001 g/l and 10 g/l
• at least 2 wt% of at least one cellulose based polymer
• at least one anionic surfactant, at least one anionic surfactant
• at least one non-ionic surfactant ,
• wherein the non-ionic surfactant is present in an amount of non-ionic surfactant to at least one cellulose based polymer in the range of between 10:1 and 1:10
• wherein the ratio of the at least one cellulose based polymer and the at least one non-ionic surfactant together to the substance is between 2:1 to 1:2,
• and wherein the concentration of the substance of a particle size between 1 nm and 999 nm and a solubility in water between 0.0001 g/l and 10 g/l is in the range of 10.1 wt% to 15 wt%.

10. A composition manufactured by the method of claim 8 comprising
• a substance of a particle size between 1 nm and 999 nm as measured by Dynamic Light Scattering (DLS) and a solubility in water between 0.0001 g/l and 10 g/l
• at least 2 wt% of at least one cellulose based polymer
• at least one anionic surfactant, at least one anionic surfactant
• at least one non-ionic surfactant ,
• wherein the non-ionic surfactant is present in an amount of non-ionic surfactant to at least one cellulose based polymer in the range of between 10: 1 and 1: 10
• wherein the ratio of the at least one cellulose based polymer and the at least one non-ionic surfactant together to the substance is between 2:1 to 1:2,
• and wherein the concentration of the substance of a particle size between 1 nm and 999 nm and a solubility in water between 0.0001 g/l and 10 g/l is in the range of 10.1 wt% to 15 wt%
for use as a medicament.

## Patentansprüche

1. Eine Zusammensetzung, umfassend
• einer Substanz mit einer Partikelgröße zwischen 1 µm und 100 µm, gemessen mittels Laserdiffraktion, und einer Löslichkeit in Wasser zwischen 0,0001 g/l und 10 g/l
• mindestens 2 Gew.-% mindestens eines Polymers auf Zellulosebasis
• mindestens ein anionisches Tensid,
• mindestens ein nichtionisches Tensid,
• wobei das nichtionische Tensid in einem Verhältnis von nichtionischem Tensid zu mindestens einem Polymer auf Zellulosebasis im Bereich von 10:1 bis 1:10 vorliegt
• wobei das Verhältnis des mindestens einen Polymers auf Zellulosebasis und des mindestens einen nichtionischen Tensids zusammen zur Substanz zwischen 2:1 und 1:2 liegt,
• wobei die Konzentration der Substanz mit einer Partikelgröße zwischen 1 µm und 100 µm und einer Wasserlöslichkeit zwischen 0,0001 g/l und 10 g/l im Bereich von 10,1 Gew.-% bis 15 Gew.-% liegt und
• wobei das mindestens eine Polymer auf Zellulosebasis aus Hydroxymethylcellulose (HPMC) und Hydroxypropylcellulose (HPC) ausgewählt ist, das mindestens eine anionische Tensid aus Natriumdodecylsulfat (SDS), Natriumdocusat (Dioctyl-Natrium-Sulfosuccinat) und Natriumoleat ausgewählt ist und das mindestens eine nichtionische Tensid ein Polyvinylpyrrolidon ist.

2. Zusammensetzung gemäß Anspruch 1, wobei in der Zusammensetzung die Substanz mit einer Partikelgröße zwischen 1 µm und 100 µm und einer Löslichkeit in Wasser zwischen 0,0001 g/l und 10 g/l als Wirkstoff vorliegt.

3. Zusammensetzung nach Anspruch 1, wobei das Polymer auf Cellulosebasis Hydroxypropylcellulose (HPC) ist und in einer Menge im Bereich zwischen 2 Gew.-% und 3 Gew.-% vorliegt.

4. Zusammensetzung nach Anspruch 1, wobei die Zusammensetzung 0,01 Gew.-% bis 10 Gew.-% eines nichtionischen Tensids mit niedrigem Molekulargewicht oder 0,01 Gew.-% bis 5 Gew.-% eines nichtionischen Tensids mit hohem Molekulargewicht umfasst.

5. Zusammensetzung nach Anspruch 4, wobei das nichtionische Tensid mit niedrigem Molekulargewicht PVP K12 ist.

6. Zusammensetzung nach Anspruch 1, wobei das nichtionische Tensid in einem Verhältnis von nichtionischem Tensid zu mindestens einem Polymer auf Cellulosebasis im Bereich zwischen 4:1 und 1:4 vorliegt.

7. Zusammensetzung gemäß Anspruch 1, wobei die Zusammensetzung besteht aus mindestens einem Wirkstoff mit einer Partikelgröße zwischen 1 µm und 100 µm, gemessen mittels Laserdiffraktion, und einer Löslichkeit in Wasser zwischen 0,001 g/l und 1 g/l, mindestens 2 Gew.-% HPC als Polymer auf Zellulosebasis, SDS als anionisches Tensid, PVP12 als nichtionisches Tensid mit niedrigem Molekulargewicht, wobei das nichtnichtionisches Tensid in einem Verhältnis von nichtionischem Tensid zu mindestens einem Polymer auf Zellulosebasis im Bereich zwischen 4:1 und 1:4 vorliegt, wobei das Verhältnis des mindestens einen Polymers auf Zellulosebasis und des mindestens einen nichtionischen Tensids zusammen zur Zusammensetzung 1:1 beträgt und wobei die Konzentration des Wirkstoffs mit einer Partikelgröße zwischen 1 µm und 100 µm und einer Löslichkeit in Wasser zwischen 0,001 g/l und 10 g/l im Bereich von 10,1 Gew.-% bis 12 Gew.-% liegt.

8. Verfahren zur Herstellung einer Zusammensetzung, die Nanopartikel umfasst, mit den folgenden Schritten:
• Bereitstellen einer Zusammensetzung gemäß Anspruch 1, die eine Substanz mit einer Partikelgröße zwischen 1 µm und 100 µm und einer Löslichkeit in Wasser zwischen 0,0001 g/l und 10 g/l, mindestens 2 Gew.-% mindestens eines Polymers auf Zellulosebasis, umfasst, mindestens ein anionisches Tensid und mindestens ein nichtionisches Tensid , wobei das mindestens eine nichtionische Tensid in einem Verhältnis von nichtionischem Tensid zu mindestens einem Polymer auf Zellulosebasis im Bereich zwischen 10:1 und 1:10 vorliegt, wobei das Verhältnis des mindestens einen Polymers auf Zellulosebasis und des mindestens einen nichtnichtionischen Tenside zusammen zu der Substanz mit einer Partikelgröße zwischen 1 µm und 1 nm im Bereich von 2:1 bis 1:2 liegt, wobei die Konzentration der Substanz im Bereich von 10,1 Gew.-% bis 15 Gew.-% liegt.
• Zerkleinerung der Zusammensetzung über einen Zeitraum, bis die Partikelgröße konstant bleibt, in einem Temperaturbereich zwischen 253 K und 323 K*)*, wodurch die Zusammensetzung, die Nanopartikel umfasst, erzeugt wird.

9. Eine nach dem Verfahren gemäß Anspruch 8 hergestellte Zusammensetzung, die somit umfasst
• eine Substanz mit einer Partikelgröße zwischen 1 nm und 999 nm, gemessen mittels dynamischer Lichtstreuung (DLS), und einer Löslichkeit in Wasser zwischen 0,0001 g/l und 10 g/l
• mindestens 2 Gew.-% mindestens eines Polymers auf Cellulosebasis
• mindestens ein anionisches Tensid, mindestens ein anionisches Tensid
• mindestens ein nichtionisches Tensid,
• wobei das nichtionische Tensid in einem Verhältnis von nichtionischem Tensid zu mindestens einem Polymer auf Zellulosebasis im Bereich zwischen 10:1 und 1:10 vorliegt
• wobei das Verhältnis des mindestens einen Polymers auf Zellulosebasis und des mindestens einen nichtionischen Tensids zusammen zur Substanz zwischen 2:1 und 1:2 liegt,
• und wobei die Konzentration der Substanz mit einer Partikelgröße zwischen 1 nm und 999 nm und einer Löslichkeit in Wasser zwischen 0,0001 g/l und 10 g/l im Bereich von 10,1 Gew.-% bis 15 Gew.-% liegt.

10. Eine nach dem Verfahren gemäß Anspruch 8 hergestellte Zusammensetzung, umfassend
• einer Substanz mit einer Partikelgröße zwischen 1 nm und 999 nm, gemessen mittels dynamischer Lichtstreuung (DLS), und einer Löslichkeit in Wasser zwischen 0,0001 g/l und 10 g/l
• mindestens 2 Gew.-% mindestens eines Polymers auf Zellulosebasis
• mindestens ein anionisches Tensid, mindestens ein anionisches Tensid
• mindestens ein nichtionisches Tensid,
• wobei das nichtionische Tensid in einem Verhältnis von nichtionischem Tensid zu mindestens einem Polymer auf Zellulosebasis im Bereich von 10:1 bis 1:10 vorliegt
• wobei das Verhältnis des mindestens einen Polymers auf Zellulosebasis und des mindestens einen nichtionischen Tensids zusammen zur Substanz zwischen 2:1 und 1:2 liegt,
• und wobei die Konzentration der Substanz mit einer Partikelgröße zwischen 1 nm und 999 nm und einer Löslichkeit in Wasser zwischen 0,0001 g/l und 10 g/l im Bereich von 10,1 Gew.-% bis 15 Gew.-% liegt
zur Verwendung als Medikament.

## Revendications

1. Une composition comprenant
• une substance dont la taille des particules est comprise entre 1 µm et 100 µm, mesurée par diffraction laser, et dont la solubilité dans l'eau est comprise entre 0,0001 g/l et 10 g/l
• au moins 2 % en poids d'au moins un polymère à base de cellulose
• au moins un agent tensioactif anionique,
• au moins un agent tensioactif non ionique,
• caratérisée en ce que l'agent tensioactif non ionique est présent dans un rapport entre l'agent tensioactif non ionique et au moins un polymère à base de cellulose compris entre 10:1 et 1:10
• **caractérisée en ce que** le rapport entre le ou les polymères à base de cellulose et le ou les agents tensioactifs non ioniques, pris ensemble, et la substance est compris entre 2:1 et 1:2,
• **caractérisée en ce que** la concentration de la substance, dont la taille des particules est comprise entre 1 µm et 100 µm et dont la solubilité dans l'eau est comprise entre 0,0001 g/l et 10 g/l, est comprise entre 10,1 % en poids et 15 % en poids, et
• caratérisée **en ce que** ledit au moins un polymère à base de cellulose est choisi parmi l'hydroxyméthylcellulose (HPMC) et l'hydroxypropylcellulose (HPC), ledit au moins un agent tensioactif anionique est choisi parmi le dodécylsulfate de sodium (SDS), le docusate de sodium (sulfosuccinate de dioctyle et de sodium) et l'oléate de sodium, et l'au moins un agent tensioactif non ionique est une polyvinylpyrrolidone.

2. Composition **caractérisée en ce que** la substance, dont la taille des particules est comprise entre 1 µm et 100 µm et dont la solubilité dans l'eau est comprise entre 0,0001 g/l et 10 g/l, constitue un principe actif.

3. Composition **caractérisée en ce que** le polymère à base de cellulose est de l'hydroxypropylcellulose (HPC) et est présent en une quantité comprise entre 2 % et 3 % en poids.

4. Composition selon la revendication 1, **caractérisée en ce que** la composition comprend de 0,01 % en poids à 10 % en poids d'un agent tensioactif non ionique de faible poids moléculaire ou de 0,01 % en poids à 5 % en poids d'un agent tensioactif non ionique de poids moléculaire élevé.

5. Composition selon la revendication 4, **caractérisée en ce que** l'agent tensioactif non ionique de faible poids moléculaire est le PVP K12.

6. Composition **caractérisée en ce que** le tensioactif non ionique est présent dans un rapport, entre le tensioactif non ionique et au moins un polymère à base de cellulose, compris entre 4:1 et 1:4.

7. Composition selon la revendication 1, **caractérisée en ce que** la composition est constituée d'au moins un principe actif dont la taille des particules est comprise entre 1 µm et 100 µm, mesurée par diffraction laser, et dont la solubilité dans l'eau est comprise entre 0,001 g/l et 1 g/l, au moins 2 % en poids de HPC en tant que polymère à base de cellulose, SDS en tant qu'agent tensioactif anionique, du PVP12 en tant qu'agent tensioactif non ionique de faible poids moléculaire, **caractérisée en ce que** l'agent tensioactif nonL'agent tensioactif non ionique est présent dans un rapport d'agent tensioactif non ionique par rapport à au moins un polymère à base de cellulose compris entre 4:1 et 1:4, **caractérisée en ce que** le rapport entre l'au moins un polymère à base de cellulose et l'au moins un agent tensioactif non ionique, pris ensemble, est de 1:1 et caratérisée **en ce que** la concentration de l'ingrédient actif, dont la taille des particules est comprise entre 1 µm et 100 µm et dont la solubilité dans l'eau est comprise entre 0,001 g/l et 10 g/l, est comprise entre 10,1 % en poids et 12 % en poids.

8. Procédé de fabrication d'une composition comprenant des nanoparticules, comprenant les étapes suivantes :
• fournir une composition selon la revendication 1 comprenant une substance dont la taille des particules est comprise entre 1 µm et 100 µm et dont la solubilité dans l'eau est comprise entre 0,0001 g/l et 10 g/l, au moins 2 % en poids d'au moins un polymère à base de cellulose, au moins un agent tensioactif anionique et au moins un agent tensioactif non ionique , caratérisée en ce que le ou les agents tensioactifs non ioniques sont présents dans un rapport entre le ou les agents tensioactifs non ioniques et le ou les polymères à base de cellulose compris entre 10:1 et 1:10, et caratérisée en ce que le rapport entre le ou les polymères à base de cellulose et le ou lesagent tensioactif non ionique, pris ensemble, par rapport à la substance présentant une taille de particules comprise entre 1 µm et 1 nm est compris entre 2:1 et 1:2, et **caractérisée en ce que** la concentration de la substance est comprise entre 10,1 % en poids et 15 % en poids.
• broyage de la composition pendant une durée suffisante pour que la taille des particules reste constante, dans une plage de températures comprise entre 253 K et 323 K, ce qui permet d'obtenir la composition comprenant des nanoparticules.

9. Une composition fabriquée selon le procédé de la revendication 8 et comprenant donc
• une substance dont la taille des particules est comprise entre 1 nm et 999 nm, telle que mesurée par diffusion dynamique de la lumière (DLS), et dont la solubilité dans l'eau est comprise entre 0,0001 g/l et 10 g/l
• au moins 2 % en poids d'au moins un polymère à base de cellulose
• au moins un agent tensioactif anionique, au moins un agent tensioactif anionique
• au moins un agent tensioactif non ionique,
• **caractérisée en ce que** le tensioactif non ionique est présent dans un rapport entre le tensioactif non ionique et au moins un polymère à base de cellulose compris entre 10:1 et 1:10
• **caractérisée en ce que** le rapport entre l'au moins un polymère à base de cellulose et l'au moins un agent tensioactif non ionique, pris ensemble, et la substance est compris entre 2:1 et 1:2,
• **caractérisée en ce que** la concentration de la substance, dont la taille des particules est comprise entre 1 nm et 999 nm et dont la solubilité dans l'eau est comprise entre 0,0001 g/l et 10 g/l, est comprise entre 10,1 % en poids et 15 % en poids.

10. Composition obtenue selon le procédé de la revendication 8, comprenant
• une substance dont la taille des particules est comprise entre 1 nm et 999 nm, mesurée par diffusion dynamique de la lumière (DLS), et dont la solubilité dans l'eau est comprise entre 0,0001 g/l et 10 g/l
• au moins 2 % en poids d'au moins un polymère à base de cellulose
• au moins un agent tensioactif anionique, au moins un agent tensioactif anionique
• au moins un agent tensioactif non ionique,
• caratérisée en ce que l'agent tensioactif non ionique est présent dans un rapport entre l'agent tensioactif non ionique et au moins un polymère à base de cellulose compris entre 10:1 et 1:10
• caratérisée en ce que le rapport entre l'au moins un polymère à base de cellulose et l'au moins un agent tensioactif non ionique, pris ensemble, et la substance est compris entre 2:1 et 1:2,
• **caractérisée en ce que** la concentration de la substance, dont la taille des particules est comprise entre 1 nm et 999 nm et dont la solubilité dans l'eau est comprise entre 0,0001 g/l et 10 g/l, est comprise entre 10,1 % en poids et 15 % en poids en vue d'une utilisation en tant que médicament.
